# EUROPEAN PATENT SPECIFICATION

(11) **EP 2 928 553 B1**
(45) Date of publication and mention of the grant of the patent: **11.01.2017**
(21) Application number: 13795517.5
(22) Date of filing: 27.11.2013
(51) Int. Cl.: A61N 1/362, A61N 1/39, A61N 1/375, A61H 31/00, A61N 1/04

(54) **EXTRACTABLE ELECTRODES FOR AUTOMATIC EXTERNAL CARDIAC DEFIBRILLATOR**
HERAUSZIEHBARE ELEKTRODEN FÜR AUTOMATISCHEN EXTERNEN DEFIBRILLATOR
ÉLECTRODES EXTRACTIBLES POUR DÉFIBRILLATEUR CARDIAQUE EXTERNE AUTOMATIQUE

(30) Priority: 04.12.2012 BE 201200819
(43) Date of publication of application: 14.10.2015
(73) Proprietor: ESM (European Safety Maintenance), 6030 Marchienne au Pont (BE)
(72) Inventor: UBAGHS, Marc, 6030 Marchienne au Pont (BE); COYETTE, Roland, 6030 Marchienne au Pont (BE); BODSON, Lucien, 4000 Liège (BE)
(74) Representative: Office Kirkpatrick
(86) International application number: PCT/EP2013/074801
(87) International publication number: WO 2014/086626

(56) References cited:
- EP-A1- 1 402 920
- US-A1- 2005 131 465
- US-A1- 2010 234 908
- US-B1- 6 178 357

## Description

### Field of the invention

The present invention concerns an automatic external cardiac defibrillator (AECD) comprising extractable electrodes that can be used by the patient himself or by a first-aider.

### Prior art

Defibrillation electrodes generally consist of a non-conductive layer produced from a flexible material, a conductive metal layer serving as an electrode and an adhesive gel, also conductive, for holding the electrode on the patient.

In order to limit phenomena of desiccation of the adhesive gel and to protect the electrode, it is usual to use an easily peelable protective film covering the adhesive gel. This is generally the case when disposable electrodes are used.

This type of electrode does however have the disadvantage of requiring numerous manipulations before being able to be applied to the patient, which may prove to be critical in the case of urgent intervention.

In order to overcome these drawbacks, electrodes with a different structure have been developed, thus simplifying use thereof. There is known through the application WO 97/26 350 a disposable electrode system for a defibrillator that is easily usable and reliable, which consists of an electrode mounted on a flexible support and held on the housing by a holding means. The electrodes are folded so that, when they are extracted, the protective film automatically detaches.

Electrodes in the form of a unitary ribbon making it possible to be positioned quickly while being simple in use are also known through US 5 191 886.

The electrodes known from the prior art are easier to use since they limit the number of steps necessary for the correct placing of the electrodes and the application of the conductive gel while preserving the integrity of the electrodes when they are not being used. However, they do not have any interaction with the AECD.

AECD devices combining several functions, which increases the chances of survival of the patient, are also known. An AECD may for example integrate audio or video communication means for obtaining assistance remotely. An AECD may also comprise location means, in particular by GPS.

Among these complementary functions, some AECDs may integrate functions facilitating cardiac resuscitation. For example, the housing of the AECD may be used advantageously as a support for cardiac resuscitation. EP 2 228 097 describes the integration of cardiopulmonary resuscitation (CPR) in the AECD housing. The dimensions and form of the AECD are adapted so that it can be placed on the chest of the patient, thus simplifying the use of the apparatus and thus enabling the first-aider to concentrate on the patient himself. The reduced dimensions of this AECD do however have the disadvantage of making its correct positioning on the sternum of the patient and its use difficult, in particular when the first-aider is inexperienced. Thus the apparatus described in EP 2 228 097 has the drawback of not being able to be kept in a stable position when the first-aider is performing CPR.

WO 2001/056 652 and US 2003/0 055 477 combine the functions of AECD and CPR, in a device that integrates the electrodes of the AECD. The use of this type of device would make it possible to obtain better stability during cardiac massage. This device, in the form of a semi-rigid mat intended to be placed on the chest of the patient in order to facilitate CPR, the mat also comprising the electrodes of the AECD. The position of the electrodes on the mat also avoids the risks of faulty placement of the electrodes. However, the electrodes must be electrically connected to an external apparatus comprising the electronic circuits necessary to the functioning of the systems situated at a distance from the mat containing the electrodes.

These systems are in fact difficult to transport and have been designed so as to be manipulated by an experienced first-aider rather than by the patient himself, who cannot use the device, which is not fully automated.

Furthermore, US2010/234908 discloses a device according to the preamble of claim 1.

There therefore exists a need for a compact and practical device combining the functions of portable AECD and CPR which is easily and rapidly usable by the patient or by a first-aider.

### Description of the invention

The invention is defined by the device according to independent claim 1.

The inventors have developed a device for solving the problem disclosed above by judicially integrating in:
- a rigid or semi-rigid housing, suitable for serving as a support for cardiopulmonary resuscitation and suitable for receiving a compression force coming from a user,
- a pair of electrodes comprising an adhesive layer.

The electrodes stored in the housing being extractable at a variable distance making it possible to obtain a total separation between the electrodes, that is to say the distance between the parts of the electrodes intended to deliver an electric shock, lying between approximately 30 and approximately 45 cm, the separation between the electrodes being maintained by locking means, preferably mechanical means generating a resistance requiring applying a traction force on the electrodes in order to extract them further. These locking means thus make it possible to ensure the correct positioning and holding of the AECD on the patient.

### Brief description of the figures

These aspects, as well as other aspects of the invention, will be clarified in the detailed description of particular embodiments of the invention, reference being made to the drawings in the figures, in which:
Figure 1 is a perspective view of the defibrillation apparatus before use.
Figure 2 is a perspective view of the defibrillator on the partial extraction of the electrodes.
Figure 3 is a perspective view of the defibrillator on complete extraction of the electrodes.
Figure 4 is longitudinal section of the defibrillator according to the invention.
Figure 5 is a view illustrating the use of the defibrillator during cardiopulmonary resuscitation.
Figure 6 shows the bottom face of the housing presenting a cruciform contact surface in contact with the patient.

### Detailed description of particular embodiments

It will appear obvious to a person skilled in the art that the present invention is not limited to the examples illustrated and described above. The invention comprises each of the novel features as well as the combination thereof. The presence of reference numbers cannot be considered to be limitative. The use of the term "comprises" may in no way exclude the presence of other elements other than those mentioned. The use of the definitive article "a" to introduce an element does not exclude the presence of a plurality of such elements. The present invention has been described in relation to specific embodiments, which have a purely illustrative value and must not be considered to be limitative.

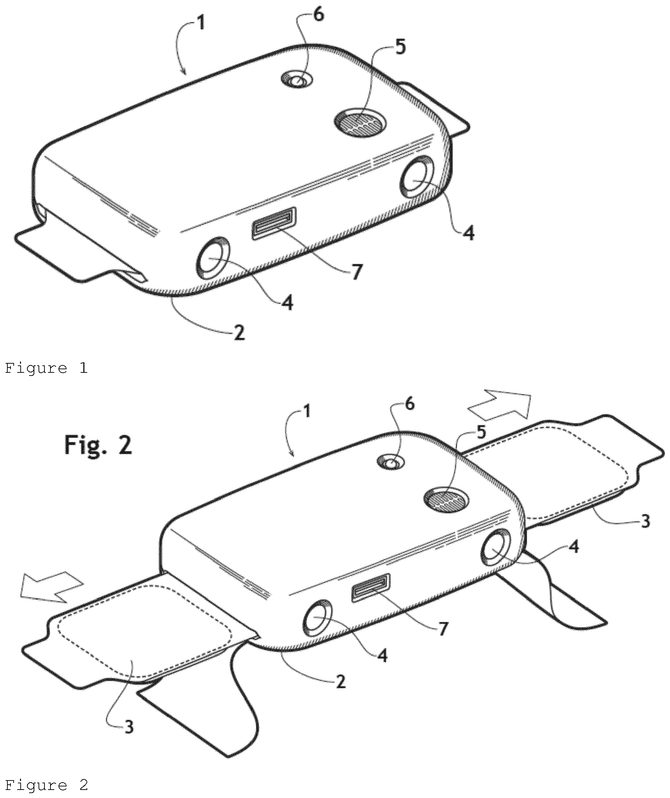

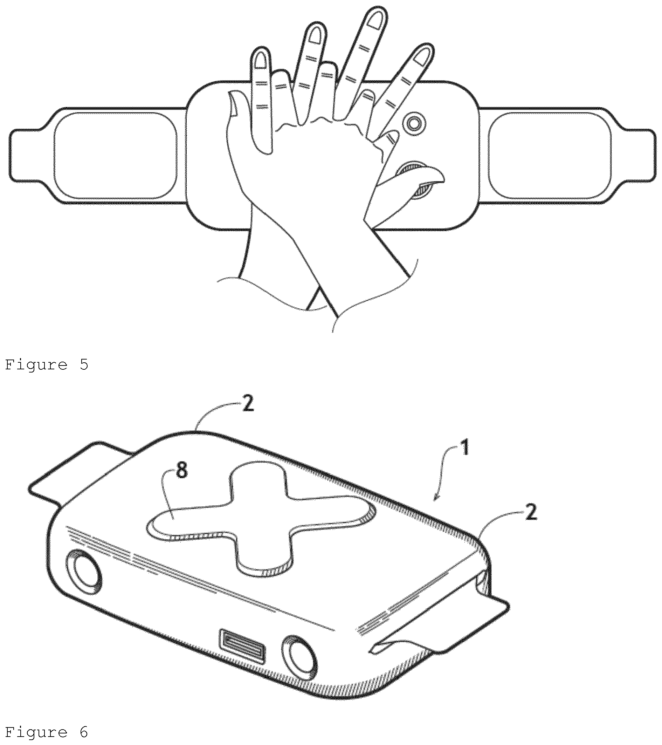

Figure 1 shows the defibrillation apparatus before use thereof, that is to say when the electrodes 3 are not yet extracted from the defibrillator. The housing 1 preferably has a length of between 15 and 20 cm and a width preferentially between 7 and 11 cm, comprises in its bottom part non-traumatic rims 2 having a rounded form. The bottom face of the housing in contact with the patient may comprise a complementary surface 8 intended to be in contact with the patient. This surface 8 concentrates the cardiac massage force on the sternum (figure 6). The opposite face of the housing 1 is suitable for a first-aider to be able to place his hands when he is performing CPR (figure 5). The surface 8 in contact with the patient is produced from an elastomer material such as rubber. According to another embodiment, the surface 8 in contact with the patient is cruciform, as shown in figure 8, thus affording better location of the force exerted by the first-aider.

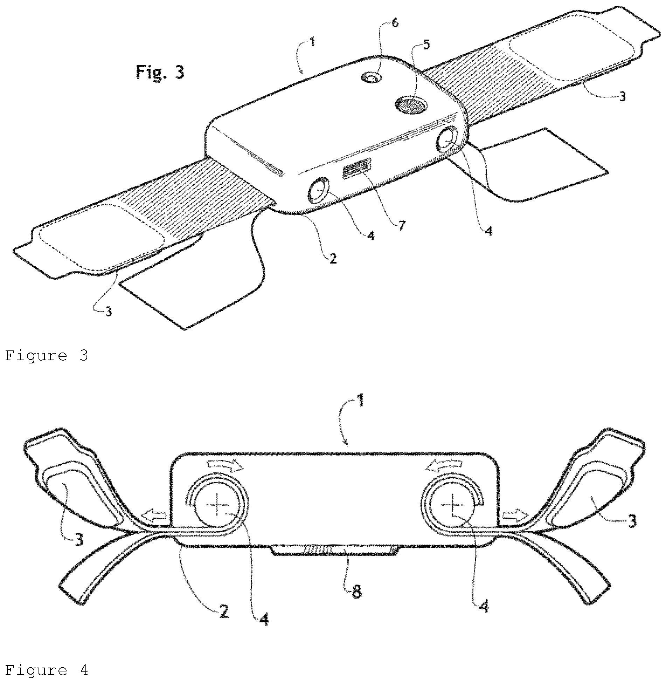

The apparatus comprises two electrodes 3 wound around an axis 4 and integrated inside the housing 1 in order to facilitate extraction thereof and extension in the case of emergency because of the absence of a cable and connections, reducing the risks of tangling and faulty connection (figure 4). Moreover, locking means generating mechanical resistance maintain the distance between the electrodes 3 during use of the AECD. The electrodes 3 have the dimensions of a ribbon with a width of around 5 to 10 cm, which also affords better positioning and holding of the housing 1 during use thereof.

The electrodes 3 comprise a substrate made from material of a polymer type covered with an adhesive layer and peelable protective film. The adhesive coating makes it possible to position and hold the electrodes 3 in position on the patient. The electrodes 3 and the housing 1 being integral, the housing 1 of the apparatus is thus directly at the level of the sternum of the patient in an ideal position for performing cardiac massage.

The separation between the electrodes 3 may advantageously be adapted according to the patient. The electrodes 3 may be in a partially extracted position, that is to say with a separation between the electrodes 3 of approximately 30 cm or completed extracted, that is to say approximately 45 cm (figures 2 and 3). An additional force is then necessary to extract the electrodes 3 beyond a separation between the electrodes 3 of approximately 30 cm. The transition between these two positions is indicated by a visual mark for distinguishing the "partial" and "total" extraction positions of the electrodes 3, indicated by hatched areas in figure 3. The change in position is also marked by an additional mechanical resistance for which an additional force will be necessary to extract the electrodes 3 further. When the electrodes 3 of the AECD are in their first position, that is to say when the electrodes 3 are partially extracted and distant from each other by approximately 30 cm, the AECD can then be utilised on patients aged more than 3 years and/or weighing less than 20 kg. When the electrodes 3 are completely extracted, that is to say separated by approximately 45 cm, the AEDC can be used on patients of more than 8 years and/or weighing more than 20 kg.

Depending on the partial or total extraction of the electrodes 3, an adapted charge is delivered; for example, in the partially extracted position, the apparatus can be used on a child of more than 3 years and/or less than 20 kg, an energy of between 60 and 80 joules two-phase, preferably around 70 joules two-phase, will be delivered. When the electrodes 3 are totally extracted, an energy of between 180 and 220 joules two-phase and preferably around 200 joules two-phase will be delivered.

The embodiment illustrated in figures 2, 3 and 4 shows, when the electrodes 3 are extracted, the automatic and simultaneous peeling of the protective film from the electrodes 3, revealing the adhesive layer and thus facilitating the rapid positioning of the electrodes 3 and the AECD.

The mechanism of extraction by unwinding is preferably synchronised and comprises a double reel, allowing the simultaneous extraction of the electrodes 3 without disturbing the positioning of the housing of the apparatus.

The external defibrillator is preferably entirely automatic and is powered up as soon as the electrodes 3 are extracted from the housing 1 and delivers an energy of approximately 70 joules two-phase. When the electrodes 3 are completely extracted, they then deliver approximately 200 joules two-phase.

Once extracted from the housing 1 and positioned on the patient through their adhesive part, the electrodes 3 make it possible to immobilise and hold the housing 1 on the apparatus on the sternum in a stable fashion. The form and position of the housing 1 facilitate any cardiac massage.

In order to be available at any time, a battery is generally chosen so that it is functional for a minimum period of one year and provides at a minimum 15 shocks with energy of 200 joules two-phase, and a continuous analysis life of at least one 1 hour.

The device may be able to provide automated assistance during the resuscitation procedure and may comprise audible 5 or visual 6 means, as well as data transfer means 7 facilitating the use of the apparatus. The audible means 5 such as a loudspeaker make it possible to use a voice guide, multifunction visual means 6 such as an indicator lamp, which may for example be green when put in service, red to alert on the electric shock or any problem and orange to indicate the end of life of the battery. The device may also comprise data transfer means 7, preferably a USB and/or WiFi port for the recovery of history data.

Optionally, this equipment will be provided for single use.

## Claims

1. Device for cardiac defibrillation, comprising:
- a rigid or semi-rigid housing (1), suitable for receiving a compression force coming from an operator,
- a pair of electrodes (3) comprising an adhesive layer,
**characterised in that** the electrodes (3) are adapted to be stored in the housing and are extractable at a variable distance making it possible to obtain a separation between the electrodes (3) of between approximately 30 cm and approximately 45 cm, the distance between the electrodes (3) being kept constant by locking means.

2. Device according to claim 1, **characterised in that** the device is adapted to produce shocks with an energy of between 60 and 80 joules two-phase at a separation between the electrodes (3) of approximately 30 cm and of 180 to 220 joules two-phase at a separation between the electrodes (3) of approximately 45 cm.

3. Device according to one of the preceding claims, **characterised in that**, when the electrodes (3) are extracted, an additional force is necessary to extract the electrodes (3) when the distance between the electrodes (3) exceeds a separation of approximately 30 cm.

4. Device according to one of the preceding claims, **characterised in that** the housing (1) comprises a proximal surface (8) in contact with the patient, produced from elastomer material.

5. Device according to one of the preceding claims, **characterised in that** the electrodes (3) are coiled inside the housing (1).

6. Device according to one of the preceding claims, **characterised in that** the electrodes (3) are coiled in the housing (1) by a double reel, allowing simultaneous extraction of the electrodes (3).

7. Device according to one of the preceding claims, **characterised in that** the housing (1) has a length of between 15 and 20 cm and a width of between 7 and 11 cm.

8. Device according to one of the preceding claims, **characterised in that**, at the time of extraction, a protective film is adapted to be removed automatically and simultaneously, revealing the adhesive layer.

9. Device according to one of the preceding claims, **characterised in that** the electrodes (3) have a width of between 5 and 10 cm.

10. Device according to one of the preceding claims, **characterised in that** the device comprises at least one audible means.

11. Device according to one of the preceding claims, **characterised in that** the device comprises at least one visual means.

12. Device according to one of the preceding claims, **characterised in that** the device comprises at least one data transfer means.

## Patentansprüche

1. Vorrichtung zur Herzdefilbrillation, die umfasst:
- ein starres oder halbstarres Gehäuse (1), das dazu geeignet ist, eine Druckkraft aufzunehmen, die von einem Benutzer ausgeübt wird,
- ein Paar Elektroden (3), umfassend eine Klebstoffschicht,
- **dadurch gekennzeichnet, dass** die Elektroden (3) so ausgelegt sind, dass sie im Gehäuse gelagert werden, und in einer variablen Strecke ausziehbar sind, wodurch möglich wird, eine Trennung zwischen den Elektroden (3) zwischen ungefähr 30 cm und ungefähr 45 cm zu erhalten, wobei die Strecke zwischen den Elektroden (3) mithilfe eines Verriegelungsmittels konstant gehalten wird.

2. Vorrichtung nach Anspruch 1, **dadurch gekennzeichnet, dass** die Vorrichtung so ausgelegt ist, Schocks mit einer Energie zwischen 60 und 80 Joule zweiphasig bei einer Trennung zwischen den Elektroden (3) von ungefähr 30 cm und von 80 bis 220 Joule zweiphasig bei einer Trennung zwischen den Elektroden (3) von ungefähr 45 cm zu erzeugen.

3. Vorrichtung nach einem der vorstehenden Ansprüche, **dadurch gekennzeichnet, dass**, wenn die Elektroden (3) herausgezogen werden, eine zusätzliche Kraft erforderlich ist, um die Elektroden (3) herauszuziehen, wenn die Strecke zwischen den Elektroden (3) eine Trennung von ungefähr 30 cm überschreitet.

4. Vorrichtung nach einem der vorstehenden Ansprüche, **dadurch gekennzeichnet, dass** das Gehäuse (1) eine proximale Oberfläche (8) umfasst, die mit dem Patienten in Berührung steht, die aus elastomerem Material hergestellt ist.

5. Vorrichtung nach einem der vorstehenden Ansprüche, **dadurch gekennzeichnet, dass** die Elektroden (3) innerhalb des Gehäuses (1) gewickelt sind.

6. Vorrichtung nach einem der vorherigen Ansprüche, **dadurch gekennzeichnet, dass** die Elektroden (3) durch eine Doppelspule im Gehäuse (1) gewickelt sind, wodurch das gleichzeitige Herausziehen der Elektroden (3) möglich wird.

7. Vorrichtung nach einem der vorstehenden Ansprüche, **dadurch gekennzeichnet, dass** das Gehäuse (1) eine Länge zwischen 15 und 20 cm und eine Breite zwischen 7 und 11 cm aufweist.

8. Vorrichtung nach einem der vorstehenden Ansprüche, **dadurch gekennzeichnet, dass** zum Zeitpunkt des Herausziehens ein Schutzfilm so ausgelegt ist, dass er automatisch und gleichzeitig entfernt wird, wodurch die Klebstoffschicht freigelegt wird.

9. Vorrichtung nach einem der vorstehenden Ansprüche, **dadurch gekennzeichnet, dass** die Elektroden (3) eine Breite zwischen 5 und 10 cm aufweisen.

10. Vorrichtung nach einem der vorstehenden Ansprüche, **dadurch gekennzeichnet, dass** die Vorrichtung zumindest ein akustisches Mittel umfasst.

11. Vorrichtung nach einem der vorstehenden Ansprüche, **dadurch gekennzeichnet, dass** die Vorrichtung zumindest ein visuelles Mittel umfasst.

12. Vorrichtung nach einem der vorstehenden Ansprüche, **dadurch gekennzeichnet, dass** die Vorrichtung zumindest ein Datenübertragungsmittel umfasst.

## Revendications

1. Dispositif pour la défibrillation cardiaque, comprenant :
- un boîtier rigide ou semi-rigide (1), adapté à recevoir une force de compression provenant d'un opérateur,
- une paire d'électrodes (3) comprenant une couche adhésive,
**caractérisé par le fait que** les électrodes (3) sont aptes à être stockées dans le boîtier et sont aptes à être extraites selon une distance variable permettant d'obtenir un écartement entre les électrodes (3) compris entre approximativement 30 cm et approximativement 45 cm, la distance entre les électrodes (3) étant maintenue constante par des moyens de blocage.

2. Dispositif selon la revendication 1, **caractérisé par le fait que** le dispositif est apte à produire des chocs avec une énergie comprise entre 60 et 80 joules biphasiques à un écartement entre les électrodes (3) d'approximativement 30 cm et de 180 à 220 joules biphasiques à un écartement entre les électrodes (3) d'approximativement 45 cm.

3. Dispositif selon l'une des revendications précédentes, **caractérisé par le fait que**, lorsque les électrodes (3) sont extraites, une force supplémentaire est nécessaire pour extraire les électrodes (3) lorsque la distance entre les électrodes (3) dépasse un écartement d'approximativement 30 cm.

4. Dispositif selon l'une des revendications précédentes, **caractérisé par le fait que** le boîtier (1) comprend une surface proximale (8) en contact avec le patient, réalisée à partir de matériau élastomère.

5. Dispositif selon l'une des revendications précédentes, **caractérisé par le fait que** les électrodes (3) sont enroulées à l'intérieur du boîtier (1).

6. Dispositif selon l'une des revendications précédentes, **caractérisé par le fait que** les électrodes (3) sont enroulées dans le boîtier (1) par un double enrouleur, permettant une extraction simultanée des électrodes (3).

7. Dispositif selon l'une des revendications précédentes, **caractérisé par le fait que** le boîtier (1) a une longueur comprise entre 15 et 20 cm et une largeur comprise entre 7 et 11 cm.

8. Dispositif selon l'une des revendications précédentes, **caractérisé par le fait qu'**au moment de l'extraction, un film protecteur est apte à être retiré de façon automatique et simultanée, révélant la couche adhésive.

9. Dispositif selon l'une des revendications précédentes, **caractérisé par le fait que** les électrodes (3) ont une largeur comprise entre 5 et 10 cm.

10. Dispositif selon l'une des revendications précédentes, **caractérisé par le fait que** le dispositif comprend au moins un moyen sonore.

11. Dispositif selon l'une des revendications précédentes, **caractérisé par le fait que** le dispositif comprend au moins un moyen visuel.

12. Dispositif selon l'une des revendications précédentes, **caractérisé par le fait que** le dispositif comprend au moins un moyen de transfert de données.
